# EUROPEAN PATENT APPLICATION

(11) **EP 2 730 560 A1**
(43) Date of publication of application: **14.05.2014**
(21) Application number: 13191499.6
(22) Date of filing: 05.11.2013
(51) Int. Cl.: C07C 269/06, C07C 271/28

(54) **Process for the preparation of an anticonvulsant compound**

(30) Priority: 12.11.2012 IT MI20121922
(71) Applicant: Dipharma Francis S.r.l., 20021 Baranzate (MI) (IT)
(72) Inventor: Attolino, Emanuele, 20021 Baranzate (MI) (IT); Rossi, Roberto, 27100 Pavia (IT); Artico, Marco, 20015 Parabiago (MI) (IT)
(74) Representative: Bertuccio, Silvia

(57) **Abstract**

The invention provides a novel method for the preparation of intermediates useful in a process designed to obtain known 1,2,4-triaminobenzene compounds, and in particular a specific compound thereof having known anticonvulsant activity. Unlike known methods, the novel method does not require advance protection of the amino groups present on the substrate.

## Description

The present invention relates to a process for the preparation of intermediates of known 1,2,4-triaminobenzene compounds with anticonvulsant activity, and in particular of a specific compound.

### PRIOR ART

The compound ethyl *N*-[2-amino-4-[(4-fluorophenyl)methylamino] phenyl]carbamate of formula **(I),** also known as retigabine, is a voltage-dependent potassium channel activator. Retigabine is used as an anticonvulsant for the treatment of epilepsy.

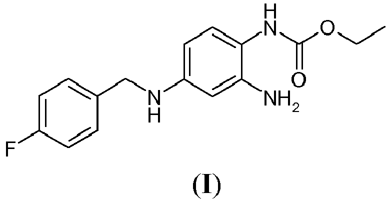

The preparation of retigabine is reported in US 5,384,330. One of the synthesis routes exemplified, as illustrated in the Scheme below, involves a series of steps comprising conversion of the functional amino group of nitroaniline of formula **A** to the corresponding ethyl carbamate, and subsequent reduction of the nitro group to obtain an aniline of formula **B.** It has to be noted that the new nitration of the aromatic ring is only conducted after protecting the amino group of the compound of formula **B** as phthalimide, to obtain a compound of formula **C.** The protection proceeds selectively in ortho to the ethyl carbamate position, to obtain a nitro-compound of formula **D.** After removal of the phthalyl protecting group from the compound of formula **D** to give the deprotected aniline of formula **E,** subsequent reductive amination with parafluorobenzaldehyde, and finally, reduction of the nitro group with Raney Ni, the retigabine of formula **(I)** is obtained

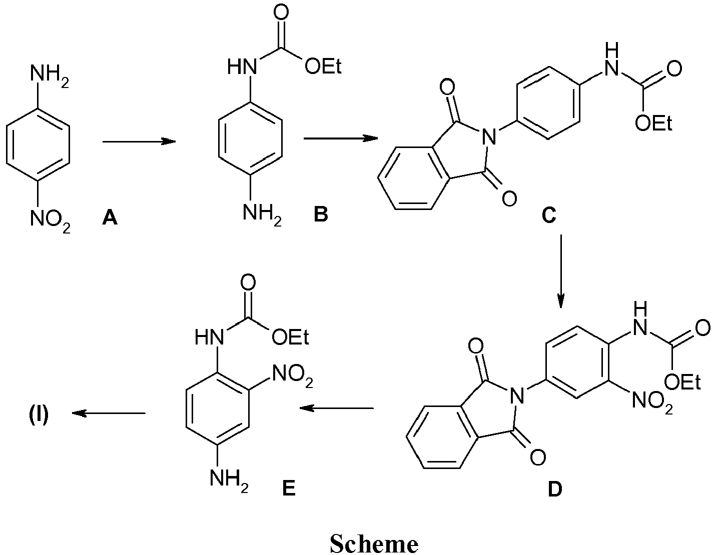

Although the process described in the Scheme is widely industrialisable and does not present any critical factors associated with the use of reagents that are hazardous to the health and the environment, it is not very efficient in terms of cost or atom economy. The synthesis is rendered onerous by the use of the protection and deprotection reactions necessary to guarantee the regioselectivity of the nitration reaction of the compound of formula **C**. This clearly lengthens the entire process by two synthesis steps, and leads to the formation of large amounts of waste water. The presence of the protecting phthalimide group in the compound of formula **C** is specific to the process, because its characteristics as a sterically hindering electron-attracting group eliminate the activating effect on aromatic electrophilic substitution of the amino group on which it is installed, especially on the ortho position. In this way, according to the method described in US 5,384,330, regioselective nitration can be obtained on the ortho position of the ethyl carbamate of formula **C**.

It should be stressed that the processes known in the prior art for the preparation of Retigabine need the use of intermediates having protected amino groups, in order to prevent the formation of undesired degradation by-products during the nitration reaction. Thus the amino groups of such intermediates are protected before the nitration reaction of the aromatic ring.

Therefore, besides the regioselectivity of the nitration of such amino intermediates, a further unsolved problem is the efficiency of the nitration reaction, to avoid the formation of undesired by-products.

There is consequently a need for a more advantageous alternative method of preparing retigabine. Said novel method should in particular be simpler and involve a few number of steps, more industrially scalable, involve the use of cheaper reagents, in particular avoid the use of protecting groups, employ mild reaction conditions, and at the same time provide retigabine with high yields, high chemical purity and efficiency.

### DESCRIPTION OF THE INVENTION

The present invention relates to a process for the preparation of a compound of formula **(II)** or a salt thereof, 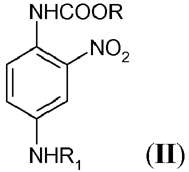 wherein R is a C₁-C₆ alkyl group and R₁ is H or an optionally substituted C₁-C₆ alkyl group, comprising the nitration reaction of a compound of formula **(III)** or a salt thereof, 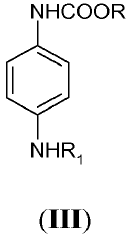 wherein R and R₁ are as defined above; and optionally, the conversion of a compound of formula (II) to another compound of formula **(II).**

According to a preferred embodiment of the invention a compound of formula **(III)** is an intermediate of formula **(IIIa),** or a salt thereof, 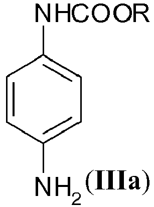 having a non-protected amino group.

A salt of a compound of formula **(II), (III)** or **(IIIa)** is, for example, a pharmaceutically acceptable salt thereof.

A C₁-C₆ alkyl group, which can be straight or branched, is preferably a C₁-C₄ alkyl group, preferably methyl, ethyl, isopropyl or tert-butyl. Said group, when substituted, can be replaced by one or more substituents, typically 1 to 3, selected independently from halogen, such as fluorine, chlorine and iodine, preferably fluorine, and phenyl, optionally substituted by one or more halogen atoms, typically 1 to 3, selected independently from halogen, such as fluorine, chlorine and iodine, preferably fluorine.

In a compound of formula **(II)** or **(III)** or **(IIIa),** substituent R is preferably ethyl.

The nitration reaction of the aromatic ring in a compound of formula **(III)** can be conducted with a nitrating reagent, typically nitric acid, for example having a concentration ranging from about 30% w/w to about 100% w/w, preferably around 65% w/w; and optionally in the presence of a solvent.

A solvent can be, for example, a polar aprotic solvent, typically an amide, such as dimethylformamide or dimethylacetamide, a chlorinated solvent, such as dichloromethane, dicloroethane, chloroform or chlorobenzene; a polar protic solvent, typically an organic or aqueous mineral acid, preferably acetic or concentrated sulphuric acid, more preferably around 95% w/w.

The nitration reaction can be conducted at a temperature of between about -10°C and the reflux temperature of the solvent, preferably between about 0°C and 25°C.

A compound of formula **(II)** can be converted to another compound of formula **(II)** according to known methods.

For example, a compound of formula **(II),** wherein R₁ is H, can be converted to a respective compound of formula **(II),** wherein R₁ is a C₁-C₆ alkyl group, as defined above, according to known methods. For example, a compound of formula **(II),** wherein R₁ is H, can be converted to another compound of formula **(II),** wherein R₁ is parafluorobenzyl, by reductive amination with parafluorobenzaldehyde, according to known methods.

It should be noted that the compounds of formula **B** and formula **E** shown in the Scheme, which summarises the synthesis method disclosed in US 5,384,330, are a compound of formula **(III)** and formula **(II)** according to the invention respectively.

It is therefore surprising that the nitration reaction of a compound of formula **(III)** and in particular **(IIIa),** effected by the process according to the invention, produces the desired compound of formula **(II)** bearing the nitro group in the ortho to the functional carbamate group position with high or practically total regioselectivity, high yields and a purity equal to or higher than 98%, measured by HPLC. Unexpectedly, contrary to the teachings of US 5,384,330, the reaction can be conducted without any use of protecting groups required to deactivate the amino function in a compound of formula **(III).**

The subject of the invention is therefore also the subsequent conversion of a compound of formula **(II),** wherein R is ethyl, thus obtained, to retigabine.

A further subject of the invention is therefore a process for the preparation of retigabine conducted, for example, according to US 5,384,330, comprising the use of an intermediate of formula **(II)** wherein R is ethyl and R₁ is hydrogen or parafluorobenzyl, obtained by the process according to the invention.

In a further aspect thereof, the invention provides a method for the preparation of retigabine comprising the use, as starting material, of a compound of formula **(II),** wherein R is ethyl and R₁ is hydrogen or parafluorobenzyl, obtained by the regioselective nitration process according to the invention.

A compound of formula **(III),** or a salt thereof, wherein R₁ is H, is known from US 5,384,330, and is commercially available. Alternatively, said compound can be prepared by reacting para-phenylenediamine of formula **(IV)** 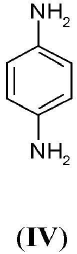 with a chloroformate of formula (Va) or a dicarbonate of formula (Vb) 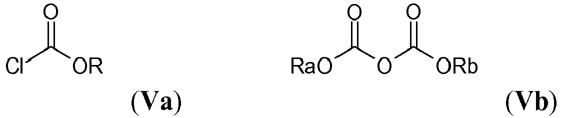 wherein, in a compound of formula **(Va),** R is as defined above; and in a compound of formula **(Vb),** Ra and Rb, which are equal or different, are as R defined above. In a compound of formula **(Va)** or formula **(Vb),** R is preferably a small alkyl. In a compound of formula **(Va),** R is typically ethyl; in a compound of formula **(Vb),** R is typically tert-butyl.

Said reaction can be conducted according to known methodologies for the formation of carbamates, for example in the presence of a base, typically a tertiary amine, and optionally of an aprotic solvent.

In a preferred aspect of the invention, a compound of formula **(III)** or a salt thereof, as defined above, wherein R₁ is H, can be prepared by reducing, by one nitro group, a compound of formula **(VI)** 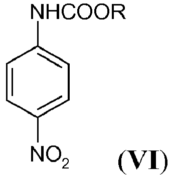 wherein R is as defined above.

The nitro group can be reduced, for example, by hydrogenation reaction with molecular hydrogen or under hydrogen transfer conditions using, for example, ammonium formate or isopropanol, in the presence of a metal catalyst, in homogenous or heterogeneous phase, for example based on Ni, Pd or Pt, optionally in the presence of chelating agents such as phosphines for catalysts in homogenous phase, or solid excipients such as charcoal for catalysts in heterogeneous phase.

A compound of formula **(III)** can be converted to another respective compound of formula **(III)** according to known methods, for example as reported above for the conversion of a compound of formula **(II)** to another compound of formula **(II).**

The reduction can also be effected with a reducing metal or a reducing metal salt in an aqueous acid environment, for example by using as reducing agent Zn, Sn or SnCl₂, Fe in the presence of dilute aqueous solutions of mineral or carboxylic acids, such as hydrochloric acid or acetic acid.

A compound of formula **(VI)** is commercially available or can be prepared in turn from nitro aniline **(VII),** which is commercially available at low cost, by reaction with a compound of formula **(Va)** or **(Vb)** as defined above, according to carbamate formation methodologies.

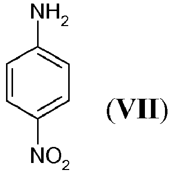

The following examples illustrate the invention:

### Example 1: Synthesis of ethyl 4-aminophenyl carbamate (IIIa R=C₂H₅)

Ethyl 4-nitrophenyl carbamate (124.5 g, 0.592 mol) is dissolved in THF (700 mL), and the solution is treated with 5% Pd/C (12.5 g, about 50% water) and ammonium formate (130.7 g). The mixture is maintained under an inert atmosphere at the temperature of about 50°C for 24 hours. When the reaction has ended, the mixture is cooled to about 25°C and filtered. The filtrate is concentrated at low pressure until a residue is obtained, which is dissolved at 50°C in toluene and washed with water. The aqueous phase is separated and the organic phase is maintained under stirring and cooled slowly to about 10°C. The white solid is filtered and stove-dried under vacuum at 50°C, and 68 g of product is obtained with a yield of 65%. A portion of the product was crystallised as hydrochloride salt from isopropanol for analysis purposes. The so obtained compound of formula **(IIIa)** has a purity of 98.7%, evaluated by HPLC.

¹H-NMR, (D₂O) of the hydrochloride salt of **(III;** R=C₂H₅): δ: 7.58 (d, 2H), 7.42 (d, 2H), 4.25 (q, 2H); 1.35 (t, 3H).

### Example 2: Synthesis of ethyl 4-amino-2-nitrophenyl carbamate (II)

Ethyl 4-aminophenyl carbamate **(IIIa),** obtained as in Example 1 (20 g, 11.1 mmol), is dissolved in a 95% w/w solution of H₂SO₄ (100 mL) and water (20 mL), and the resulting mixture is maintained under stirring and cooled to -5°C. 65% w/w HNO₃ (8.5 mL) is added slowly, and the reaction mixture is maintained under stirring in an inert atmosphere at 0°C for 30 minutes. The end-of-reaction mixture is then quenched by slow dripping in water (500 mL) at the temperature of 5°C. The solid formed is filtered, and 15 g of product is obtained with complete selectivity, yield of 60%, and a purity of 98.6%, evaluated by HPLC.

¹H-NMR, (CDCl₃) δ: 9.35 (s, 1H, NH), 8.26 (d, *J* = 9.0 Hz, 1H), 7.43 (d, *J* = 2.7 Hz, 1H), 6.96 (dd, *J* = 9.0 e 2.7 Hz, 1H), 4.22 (q, 2H); 1.32 (t, 3H).

### Example 3: Synthesis of ethyl N-[2-amino-4-[(4-fluorophenyl) methylamino]phenyl]carbamate (I)

Glacial acetic acid (204 g) is added by slow dripping to a suspension of NaBH₄ (41 g) in THF (500 mL), maintaining the temperature at around 25°C. At the end of the addition the mixture is left under stirring for 15 minutes and then treated with ethyl 4-amino-2-nitrophenyl carbamate **(II)** (49 g, 21.8 mmol), prepared as described in Example 2, and the mixture is heated to 35°C. 4-fluorobenzaldehyde is then added by slow dripping, and an hour after the end of the addition the mixture is quenched by slow addition of water (370 mL) and solid K₂CO₃ (100 g), added in portions. The mixture is maintained under stirring for 15 minutes; the phases are then separated and the organic phase is concentrated at low pressure, by co-evaporating with toluene. 95 g of crude product is obtained, part of which is purified for the purpose of analytical characterisation. The desired compound for formula **(I)** has a purity higher than 99% measured by HPLC.

¹H-NMR, (CDCl₃) δ: 9.35 (s, 1H, NH), 8.26 (d, *J* = 9.0 Hz, 1H), 7.29 (m, 3H), 7.03 (t, 2H), 6.95 (dd, *J* = 9.0 e 2.7 Hz, 1H), 4.30 (s, 2H), 4.21 (q, 2H); 1.30 (t, 3H).

80 g of intermediate is dissolved in ethanol (640 mL), and the solution is acidified by adding 36% HCl (160 mL). Iron powder is added (32.2 g), and the mixture is maintained under stirring for one hour at about 25°C. The end-of-reaction mixture is then filtered, and the solid obtained is divided between a dilute aqueous solution of K₂CO₃ and ethyl acetate. The phases are separated and the organic phase is concentrated at low pressure to obtain a residue which, when taken up with isopropanol (50 mL), leads to the formation of an abundant solid. The mixture is then filtered, and the product obtained is recrystallised from isopropanol (90 mL). When the crystals have been stove-dried under vacuum at 40°C, 24.6 g of product is obtained, with chemico-physical and spectroscopic characteristics identical to those reported for compound (I) in Die Pharmazie 1998, 53, 865-869.

## Claims

1. A process for preparing a compound of formula **(II),** or a salt thereof, 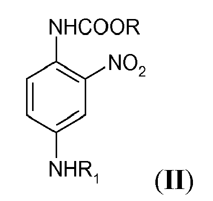 wherein R is a C₁-C₆ alkyl group, and R₁ is H or an optionally substituted C₁-C₆ alkyl group, comprising nitrating a compound of formula **(III),** or a salt thereof, 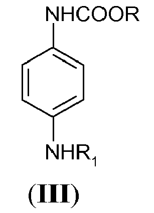 wherein R and R₁ are as defined above; and optionally converting a compound of formula **(II)** to another compound of formula **(II).**

2. A process according to claim 1, for preparing a compound of formula **(II),** wherein R₁ is H, or a salt thereof, comprising nitrating a compound of formula **(IIIa)** or a salt thereof, 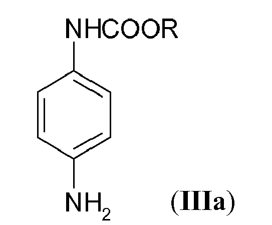 wherein the amino group is not protected and R is as defined in claim 1.

3. A process according to claim 1 or 2, wherein in a compound of formula **(II), (III)** or **(IIIa)** the substituent R is ethyl.

4. A process according to claim 1, wherein the nitrating reaction is carried out by using a nitrating agent, typically nitric acid having a concentration from about 30 % w/w to about 100% w/w.

5. Process according to claim 4, wherein nitric acid has a concentration of about 65% w/w.

6. A process according to claim 4 or 5, wherein the nitrating reaction is carried out in the presence of a solvent.

7. A process according to claim 6, wherein the solvent is a polar aprotic solvent, a chlorinated solvent, a polar protic solvent, typically an aqueous organic or a mineral acid.

8. A process according to claim 7, wherein the mineral acid is acetic acid or concentrated sulfuric acid.

9. A process according to claim 8, wherein the mineral acid is sulfuric acid having a concentration of about 95% w/w.

10. A process according to claim 1, wherein the nitrating reaction is carried out at a temperature ranging from about -10°C and the solvent reflux temperature, preferably between about 0°C and 25°C.

11. A process according to each of claims 1 to 10, comprising the subsequent conversion of a compound of formula **(II),** or a salt thereof, wherein R is ethyl into Retigabine of formula **(I)**

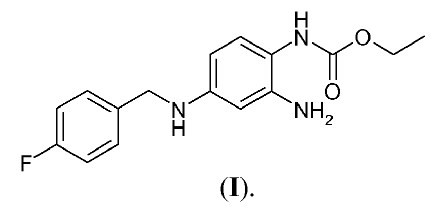

12. A process according to claim 11, wherein in a compound of formula **(II),** or a salt thereof, R is ethyl and R₁ is H.

13. A process for preparing Retigabine of formula **(I)** 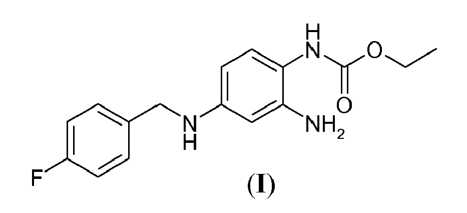 comprising using an intermediate compound of formula **(II),** or a salt thereof, 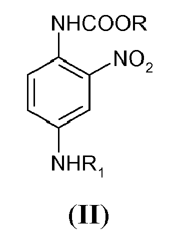 wherein R₁ is H obtained according to the process of each of the claims from 1 to 11.

14. A process according to claims 1-12, further comprising the preparation of a compound of formula **(III),** or a salt thereof, wherein R is a C₁-C₆ alkyl group and R₁ is H by reducing the nitro group in a compound of formula **(VI)** 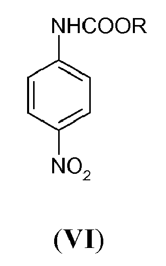 wherein R is as defined above.

15. A process according to claim 14, wherein in a compound of formula **(VI)** R is ethyl.
